# EUROPEAN PATENT APPLICATION

(11) **EP 2 606 857 A1**
(43) Date of publication of application: **26.06.2013**
(21) Application number: 13160460.5
(22) Date of filing: 26.10.2010
(51) Int. Cl.: A61F 2/38, A61F 2/42

(54) **Prosthesis with composite component**

(30) Priority: 30.10.2009 US 256517 P; 17.11.2009 US 620034
(62) Divisional of application: 10188965.7
(71) Applicant: DePuy Products, Inc., Warsaw, IN 46581 (US)
(72) Inventor: Deffenbaugh, Daren L, Warsaw, IN Indiana 46590 (US); Wogoman, Thomas E, Warsaw, IN Indiana 46580-8742 (US)
(74) Representative: Belcher, Simon James

(57) **Abstract**

An ankle prosthesis comprises a bearing (216), a talar component (212), and a distal tibial component (214) having a (i) proximal bone-engaging surface (226), (ii) a distal mounting surface (220), and (iii) an extension (224) extending from the proximal bone-engaging surface to a proximal end (230) along an axis intersecting the proximal surface. The proximal exterior surface extends proximally extension from the proximal end for at least part of the axial length of the extension. The distal tibial component comprises a composite including a solid polymer portion (220) and a porous portion (222). The solid polymer portion defines the proximal end of the extension and the mounting surface of the distal tibial component and bears against the mounting surface of the bearing. The porous portion of the tibial component defines the proximal bone-engaging surface of the tibial component and the proximal exterior surface of the extension.

## Description

The present invention relates generally to an implantable orthopaedic prosthesis, and more particularly to an implantable prosthesis having a bearing component and another component supporting the bearing component.

During the lifetime of a patient, it may be necessary to perform a joint replacement procedure on the patient as a result of, for example, disease or trauma. The joint replacement procedure may involve the use of a prosthesis that is implanted into one or more of the patient's bones. In the case of a knee replacement procedure, a tibial tray is implanted into the patient's tibia. A bearing is then secured to the tibial tray. The condyle surfaces of a replacement femoral component bear against the tibial bearing.

One type of knee prosthesis is a fixed-bearing knee prosthesis. As its name suggests, the bearing of a fixed-bearing knee prosthesis does not move relative to the tibial tray. Fixed-bearing designs are commonly used when the condition of the patient's soft tissue (i.e. knee ligaments) does not allow for the use of a knee prosthesis having a mobile bearing.

In contrast, in a mobile-bearing type of knee prosthesis, the bearing can move relative to the tibial tray. Mobile-bearing knee prostheses include so-called "rotating platform" knee prostheses, in which the bearing can rotate about a longitudinal axis on the tibial tray.

Tibial trays are commonly made of a biocompatible metal, such as a cobalt chromium alloy, stainless steel or a titanium alloy. Solid forms of these materials have an elastic modulus (Young's modulus) substantially greater than that of natural bone. For example, as disclosed in US-A-2009/192610, cobalt chromium alloy has been reported to have an elastic modulus of 220 GPa (gigapascals) and titanium alloy 6A1 4V has been reported to have an elastic modulus of 110 GPa. This document also reports that the elastic modulus of cortical bone is 15 GPa and the elastic modulus of trabecular bone is 0.1 GPa. When a tibial tray made of cobalt chromium alloy or titanium alloy is assembled with a bearing made of, for example, ultrahighmolecular weight polyethylene (UHMWPE), the total construct, including the tibial tray and the bearing, may have an effective stiffness that can result in non-optimal load transfer between the tibial implant construct and the underlying bone of the proximal tibia: stress shielding may occur in some areas of the proximal tibia, resulting in bone resorption and implant loosening.

For both fixed and mobile-bearing knee prostheses, the tibial trays may be designed to be cemented into place on the patient's tibia or alternatively may be designed for cementless fixation. Cemented fixation relies on mechanical bonds between the tibial tray and the cement as well as between the cement and the bone. Cementless implants generally have surface features that are conducive to bone ingrowth into the implant component, and rely to a substantial part, on this bony ingrowth for fixation.

Tibial components of both fixed and mobile-bearing and cemented and cementless knee arthroplasty systems are commonly modular components, comprising a tibial tray and a polymeric bearing carried by the tibial tray. The tibial trays commonly include features extending distally, such as pegs or stems. These extensions penetrate below the surface of the tibial plateau and stabilize the tibial tray component against movement. In cementless tibial implants, the outer surfaces of these extensions are typically porous to allow for bone ingrowth. For example, in the Zimmer Trabecular Metal Monoblock tibial trays, pegs with flat distal surfaces and hexagonal axial surfaces are formed completely of a porous metal. In such trays, bone ingrowth is likely to occur along all surfaces of the pegs, including the distal surfaces.

On occasion, the primary knee prosthesis fails. Failure can result from many causes, including wear, aseptic loosening, osteolysis, ligamentous instability, arthrofibrosis and patellofemoral complications. When the failure is debilitating, revision surgery may be necessary. In a revision, the primary knee prosthesis (or parts of it) is removed and replaced with components of a revision prosthetic system.

When the tibial implant includes extensions (such as pegs or stems) that extend into the natural bone, a revision surgery usually requires a large resection of the bone in order to dislodge the extensions from the bone. This large resection not only complicates the surgery, it also requires removal of more of the patient's natural bone than is desirable. This removal of additional bone may further compromise the bone, increase the risk of onset of bone pathologies or abnormalities, or reduce the available healthy bone for fixation of the revision implant. Moreover, the large resection usually means that a larger orthopaedic implant is necessary to fill the space and restore the joint component to its expected geometry.

This difficulty in dislodging the tibial tray from the bone is worsened by the fact that bone also grows into the distal surfaces of the extensions. Severing these connections is problematic since these areas are not easily accessible from the tibial plateau.

Similar issues may be presented in other types of joint prostheses.

The invention provides an ankle prosthesis comprising:
a distal tibial component having a (i) proximal bone-engaging surface, (ii) a distal mounting surface, and (iii) an extension extending from the proximal bone-engaging surface to a proximal end along an axis intersecting the proximal surface, the extension having an axial length and an exterior surface including a distal exterior surface adjacent to the proximal bone-engaging surface of the distal tibial component and a proximal exterior surface, the proximal exterior surface proximally extending from the proximal end for at least part of the axial length of the extension,
a bearing having an articulation surface and a mounting surface, and
a talar component having an articulation surface and a bone-engaging surface,
in which:
   the distal tibial component comprises a composite including a solid polymer portion and a porous portion,
   the solid polymer portion of the distal tibial component defines the proximal end of the extension and the mounting surface of the distal tibial component and bears against the mounting surface of the bearing,
   the solid polymer portion of the distal tibial component is secured to the porous portion of the tibial component, and
   the porous portion of the tibial component defines the proximal bone-engaging surface of the tibial component and the proximal exterior surface of the extension.

Optionally, the porous portion defines the distal exterior surface of the extension.

Optionally, the solid polymer portion comprises polyetheretherketone (PEEK).

Optionally, the solid polymer portion comprises fibre-reinforced PEEK.

Optionally, the bearing comprises a polymer material different from the solid polymer portion of the distal tibial component.

Optionally, the bearing comprises UHMWPE and the solid polymer portion of the distal tibial component comprises fibre-reinforced PEEK.

The invention also provides a joint prosthesis comprising:
a first component having an articulation surface,
a bearing having an articulation surface shaped to bear against the articulation surface of the first component and an opposite surface, and
a composite component having a mounting surface, a bone-engaging surface and an extension extending out from the bone-engaging surface opposite the mounting surface,
in which:
   the opposite surface of the bearing and the mounting surface of the composite component have complementary locking features for mounting the bearing on the composite component,
   the extension is configured for stabilizing the composite component when implanted in a bone of a patient,
   the extension has an end opposite from the mounting surface of the composite component,
   the composite component includes a porous portion and a solid portion,
   the solid portion defines the mounting surface of the composite component and the end of the extension,
   the porous portion defines the bone-engaging surface of the composite component,
   the solid portion comprises a polymer, and
   the solid portion and the porous portion are bonded together.

Optionally, the prosthesis is a knee prosthesis, and the first component comprises a distal femoral implant component and the composite component comprises a tibial tray.

Optionally, the prosthesis is an ankle prosthesis.

Optionally, the solid portion of the composite component comprises PEEK.

Optionally, the solid portion of the composite component comprises fibre-reinforced PEEK.

Optionally, the porous portion of the composite component comprises metal foam, for example titanium foam.

Optionally, the porous portion of the composite comprises polymer foam, for example PEEK foam.

Optionally, the porous portion of the composite comprises fibre-reinforced PEEK foam.

Optionally, the composite component includes a metal plate positioned between the mounting surface and the bone-engaging surface.

Optionally, the metal plate includes a cut-out and in which polymer extends through the cut-out of the metal plate.

Optionally, the cut-out comprises a through-bore, the metal plate including a plurality of through-bores and the polymer extending through the through-bores.

Optionally, the polymer extending through the through-bores comprises polymer foam.

Optionally, the polymer extending through the through-bores comprises solid polymer.

In another aspect, the invention provides a joint prosthesis comprising:
a first component having an articulation surface,
a bearing having an articulation surface shaped to bear against the articulation surface of the first component and an opposite surface, and
a composite component having a mounting surface, a bone-engaging surface and an extension extending out from the bone-engaging surface opposite the mounting surface,
in which:
   the opposite surface of the bearing and the mounting surface of the composite component have complementary locking features for mounting the bearing on the composite component,
   the extension is configured for stabilizing the composite component when implanted in a bone of a patient,
   the composite component includes a porous polymer portion, a solid polymer portion and a metal reinforcing portion,
   the solid polymer portion defines the mounting surface of the composite component,
   the porous polymer portion defines the bone engaging surface of the composite component, and
   the metal reinforcing portion is positioned between and spaced from the mounting surface and the bone-engaging surface.
   the solid polymer portion, porous polymer portion and metal reinforcing portion are bonded together.

Optionally, the metal reinforcing portion comprises a plate having a cut-out and in which polymer extends through the cut-out from the solid polymer portion to the porous polymer portion.

Optionally, the cut-out comprises a through-bore.

Optionally, the extension comprises solid polymer integral with the solid polymer portion defining the mounting surface.

Optionally, the polymer extending through the through-bore comprises porous polymer.

In another aspect, the present invention provides a knee prosthesis comprising a femoral component, a bearing and a tibial tray. The femoral component has a medial condyle surface and a lateral condyle surface. The bearing has a distal surface and a proximal surface. The proximal surface includes (i) a medial bearing surface configured to articulate with the medial condyle surface of the femoral component, and (ii) a lateral bearing surface configured to articulate with the lateral condyle surface of the femoral component. The bearing is secured to the tibial tray. The tibial tray has a platform having (i) a proximal surface, (ii) a distal surface opposite the proximal surface, and (iii) an extension extending from the distal surface of the platform to a distal end along an axis intersecting the distal surface. The extension has an axial length and an exterior surface including a proximal exterior surface adjacent to the distal surface of the platform and a distal exterior surface. The distal exterior surface extends proximally from the distal end for at least part of the axial length of the extension. The tibial tray comprises a composite including a solid polymer portion and a porous portion. The solid polymer portion of the tibial tray defines the proximal surface of the platform and bears against the distal surface of the bearing. The polymer portion extends from the proximal surface of the platform into the extension and defines the distal exterior surface of the extension. The solid polymer portion is secured to the porous portion of the tibial tray. The porous portion of the tibial tray has a greater porosity than the femoral component. The porous portion defines the distal surface of the platform.

Optionally, the porous portion defines the proximal exterior surface of the extension.

Optionally, the distal exterior surface of the extension is generally spheroidal.

Optionally, the polymer portion comprises polyetheretherketone (PEEK).

Optionally, the polymer portion comprises fibre-reinforced PEEK.

Optionally, the bearing comprises a polymer material different from the polymer portion of the tibial tray.

Optionally, the bearing comprises UHMWPE and the polymer portion of the tibial tray comprises fibre-reinforced PEEK.

Optionally, the extension comprises a stem.

Optionally, the extension comprises a peg.

Optionally, the tibial tray includes a plurality of spaced pegs. In such embodiments, each peg may extend from the distal surface of the platform to a distal end along an axis intersecting the distal surface. Each of the pegs may have an axial length and an exterior surface including a proximal exterior surface adjacent to the distal surface of the platform and a distal exterior surface intersected by the axis of the extension and spaced from the platform. In such embodiments, the polymer portion may extend from the proximal surface of the platform into each peg and may define the distal exterior surface of each peg. In such embodiments, the porous portion may extend from the distal surface of the platform in a distal direction and defines the proximal exterior surface of each peg.

Optionally, the prosthesis is a fixed-bearing prosthesis, and the distal surface of the bearing and the proximal surface of the tibial tray platform include complementary locking features. In such embodiments, the locking features of the proximal surface of the tibial tray platform may be formed in the polymer portion of the tibial tray platform.

In another aspect, the present invention provides a method of making a tibial tray component of a knee prosthesis. A porous base is provided. The base has a proximal surface, a distal surface and an extension extending distally from the proximal surface to a distal end. The extension has an opening at the distal end. The porous base has an interior surface extending from the proximal surface to the opening at the distal end of the extension. A quantity of fibre-reinforced PEEK material is provided. The fibre-reinforced PEEK material is moulded to the porous base so that the fibre-reinforced PEEK overlies the proximal surface of the porous base and so that the fibre reinforced PEEK material is moulded to the interior surface of the porous base and extends distally out of the opening at the distal end of the extension.

Optionally, the moulding step comprises injection moulding.

Optionally, the step of moulding the fibre-reinforced polyetheretherketone (PEEK) material to the porous base includes forming a locking mechanism in the polyetheretherketone (PEEK) material that is moulded to the proximal surface of the porous base.

Optionally, the method includes the step of placing a reinforcing plate on the porous base before moulding the fibre-reinforced PEEK material to the porous base and in which the step of moulding the fibre-reinforced PEEK material to the porous base holds the metal plate on the base.

The present invention addresses the need for a prosthesis with a modular implant component suitable for cementless fixation that can be removed more readily from the bone in revision surgery to conserve native bone. The present invention also addresses the need for an implant with an effective stiffness less than that of an implant construct using tibial trays made of conventional solid titanium alloy and cobalt chromium alloy. Examples of joint prostheses to which the invention is applicable include for example knee joint prostheses and ankle joint prostheses.

Embodiments of the invention are described below by way of example with reference to the accompanying drawings, in which:
FIG. 1 is an exploded perspective view of a fixed-bearing knee prosthesis,
FIG. 2 is a bottom perspective view of the bearing of the prosthesis of FIG. 1,
FIG. 3 is a perspective view of the tibial tray of the knee prosthesis of FIG. 1,
FIG. 4 is a bottom plan view of the tibial tray of the knee prosthesis of FIG. 1,
FIG. 5 is a cross sectional view of the tibial tray of FIGS. 1 and 4 taken along the line 5-5 of FIG. 4, as viewed in the direction of the arrows,
FIG. 6 is a perspective view of the porous portion of the tibial tray of the knee prosthesis of FIGS. 1, 4 and 5, shown as a preform prior to being moulded to form the tibial tray illustrated in FIGS. 1, 4 and 5,
FIG. 7 is a bottom plan view of the porous preform of FIG. 6,
FIG. 8 is a cross sectional view of the porous preform of FIGS. 6 and 7, taken along line 8-8 of FIG. 7, as viewed in the direction of the arrows,
FIG. 9 is a perspective view of an alternative embodiment of a preform,
FIG. 10 is a bottom plan view of the preform of FIG. 9,
FIG. 11 is a cross sectional view of the preform of FIGS. 9 and 10, taken along lines 11-11 of FIG. 10, as viewed in the direction of the arrows,
FIG. 12 is a cross sectional view similar to FIG. 5, showing a tibial tray made from the preform of FIGS. 9 to 11,
FIG. 13 is a cross sectional view similar to FIGS. 5 and 12, showing a tibial tray made from another embodiment of a preform,
FIG. 14 is a schematic cross sectional view of the interface of the porous portion and the polymer portion of the tibial tray,
FIG. 15 is a cross sectional view similar to FIGS. 5, 12 and 13, showing the tibial tray of FIGS. 1 and 3 to 5 assembled with a femoral component and a bearing,
FIG. 16 is a perspective view of an ankle prosthesis,
FIG. 17 is a cross-sectional view, similar to the view of FIG. 5, of another embodiment of a tibial tray,
FIG. 18 is a perspective view of the metal plate portion of the tibial tray of FIG. 17,
FIG. 19 is a bottom plan view of the metal plate of FIG. 18,
FIG. 20 is a perspective view of the porous portion of the tibial tray of FIG. 17,
FIG. 21 is a bottom plan view of the porous portion of the tibial tray of FIGS. 17
   and 20, and
FIG. 22 is a cross-sectional view of the porous portion of the tibial tray of FIGS. 17 and 20-21, taken along line 22-22 of FIG. 21, viewed in the direction of the arrows.

Terms representing anatomical references, such as anterior, posterior, medial, lateral, superior, inferior etc may be used throughout this specification in relation to both the orthopaedic implants described herein and a patient's natural anatomy. Such terms have well-understood meanings in both the study of anatomy and the field of orthopaedics. Use of such anatomical reference terms in the specification is intended to be consistent with their well-understood meanings unless noted otherwise.

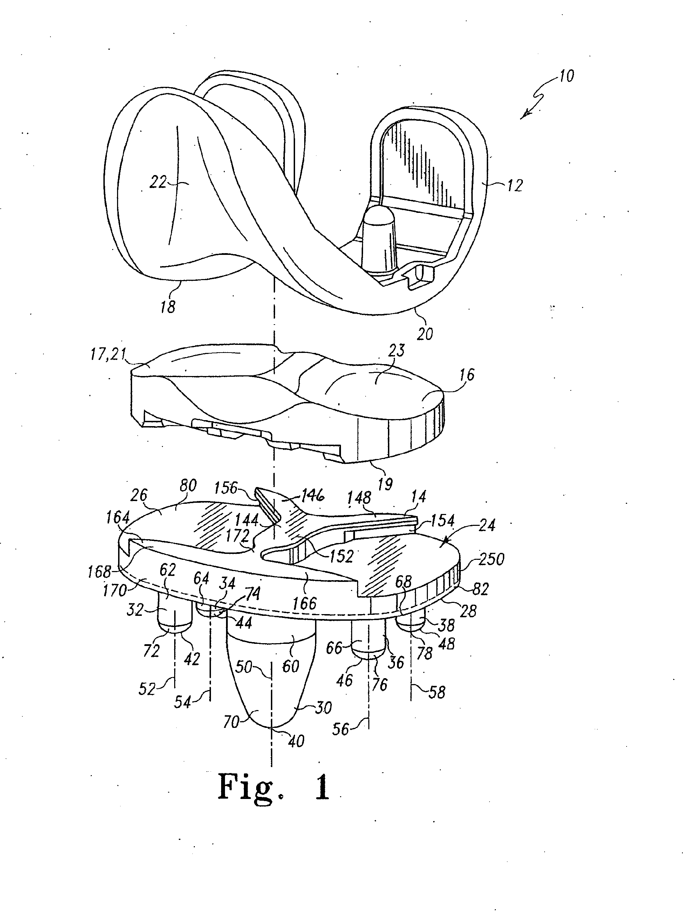

Referring to the drawings, FIG. 1 shows a knee prosthesis 10 which includes a femoral component 12, a tibial tray 14, and a bearing 16. The knee prosthesis 10 is a fixed bearing knee prosthesis, meaning that no movement is intended to occur between the tibial tray 14 and the bearing 16.

The femoral component 12 includes two condylar bearing surfaces: a medial condyle surface 18 and a lateral condyle surface 20. The femoral component 12 is configured to be implanted into a surgically prepared end of the patient's femur (not shown), and is configured to emulate the configuration of the patient's natural femoral condyles. As such, the lateral condyle surface 20 and the medial condyle surface 18 are configured (e.g., curved) in a manner which mimics the condyles of the natural femur. The lateral condyle surface 20 and the medial condyle surface 18 are spaced apart from one another thereby defining an intercondylar notch 22 between them. The intercondylar notch 22 defines a patella groove shaped to receive and bear against a patella implant component (not shown). The femoral component 12 of FIG. 1 is a cruciate retaining component, although it should be understood that the principles of the present invention are applicable to cruciate substituting prosthetic knee systems as well. The femoral component 12 may include features of standard, commercially available implants, such as those available from DePuy Orthopaedics, Inc., Warsaw, Indiana, as well as those available from other suppliers of prosthetic knee systems. The femoral component 12 may also include features disclosed in US-A-2010/036500, US-A-2009/032664, US-A-2009/0326667, US-A-2009/326666, and US-A-2009/326665.

The femoral component 12 may be constructed from a biocompatible metal, such as stainless steel, titanium, cobalt chromium alloy or titanium alloy, although other materials may also be used. The bone-engaging surfaces of these components may include cement pockets to facilitate cementing the component to the bone. The bone-engaging surfaces of the femoral component may alternatively be porous to promote bone ingrowth for permanent fixation.

As shown in FIG. 1, the bearing component 16 has a proximal articulation surface 17 and a distal mounting surface 19 opposite the proximal articulation surface 17. The proximal articulation surface 17 of the bearing 16 includes a medial bearing surface 21 configured to articulate with the medial condyle 18 of the femoral component 12 and a lateral bearing surface 23 configured to articulate with the lateral condyle 20 of the femoral component 12. The bearing component 16 is modular, and is assembled with the tibial tray 14 intraoperatively and secured thereto through a mechanical interlocking mechanism, as described in more detail below.

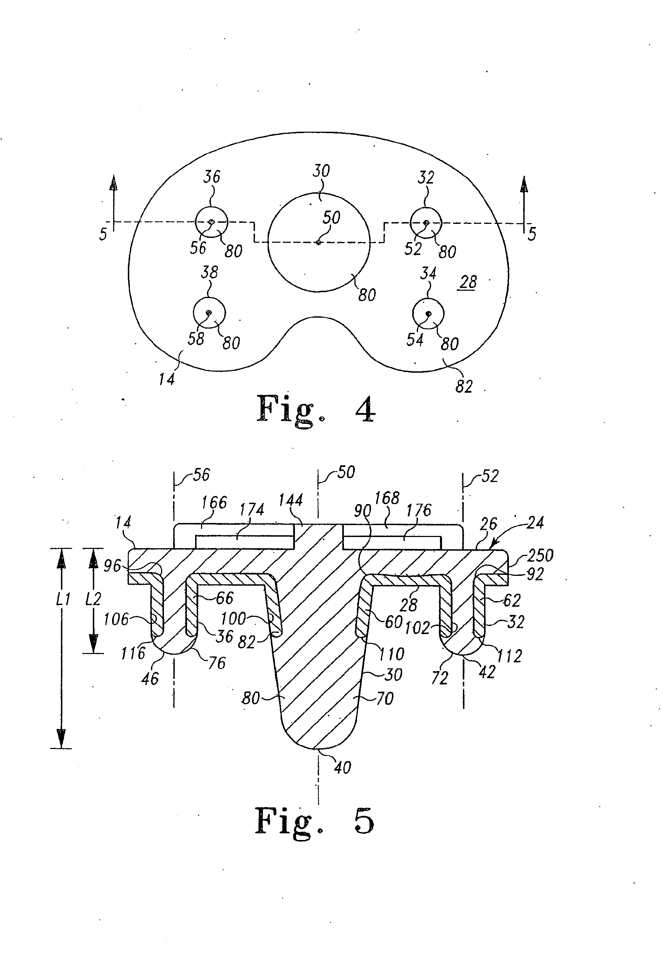

The tibial tray 14 includes a platform 24 having a proximal mounting surface 26 and an opposite distal bone-engaging surface 28. The illustrated tibial tray 14 also includes a plurality of extensions 30, 32, 34, 36, 38 extending distally from the distal bone-engaging surface 28 of the platform to distal ends 40, 42, 44, 46, 48 along axes 50, 52, 54, 56, 58 intersecting the distal surface 28 of the platform 24. Each extension 30, 32, 34, 36, 38 has an axial length, shown, for example, as L₁ and L₂ in FIG. 5. Each extension 30, 32, 34, 36, 38 also has an exterior surface comprising a proximal exterior surface 60, 62, 64, 66, 68 adjacent to the distal surface 28 of the tibial platform 24 and a distal exterior surface 70, 72, 74, 76, 78 at the distal ends 40, 42, 44, 46, 48 of the extensions. The distal exterior surfaces 70, 72, 74, 76, 78 of the extensions 30, 32, 34, 36, 38 extend proximally from the distal ends 40, 42, 44, 46, 48 of the extensions at least part of the axial length of each extension 30, 32, 34, 36, 38. The distal exterior surfaces 70, 72, 74, 76, 78 of the stem 30 and pegs 32, 34, 36 38 are generally spheroid-shaped in the device shown in this drawing.

The tibial tray 14 is a composite of two materials, including a solid polymer portion 80 and a porous portion 82. As used herein, "solid polymer" is used to identify a material that lacks void space, having substantially 100% of theoretical density. As used herein, "porous portion" is used to identify a portion of the implant made of a material that has void space and that is less than 100% of theoretical density. As discussed in more detail below, the porous portion may comprise a biocompatible metal or a biocompatible polymer.

The solid polymer portion 80 of the tibial tray 14 defines the proximal surface 26 of the platform 24 and bears against the distal surface 19 of the bearing component 16 when assembled. The solid polymer portion 80 extends from the proximal surface 26 of the platform into each of the extensions 30, 32, 34, 36 38, and defines the distal exterior surfaces 70, 72, 74, 76, 78 of the extensions 30, 32, 34, 36, 38.

The polymer portion 80 of the tibial tray 14 is secured to the porous portion 82, as described in more detail below.

The porous portion 82 of the tibial tray defines the distal bone-engaging surface 28 of the platform 24. This porous distal surface 28 faces the bone of the resected proximal surface of the tibial plateau, and defines a material that is conducive to bone ingrowth to allow for uncemented fixation of the tibial platform to the proximal tibia. As described in more detail below, the porous portion 82 extends proximally from the distal surface 28 and intermeshes with the solid polymer portion 80 at a location between the distal surface 28 and the proximal surface 26 of the platform 24.

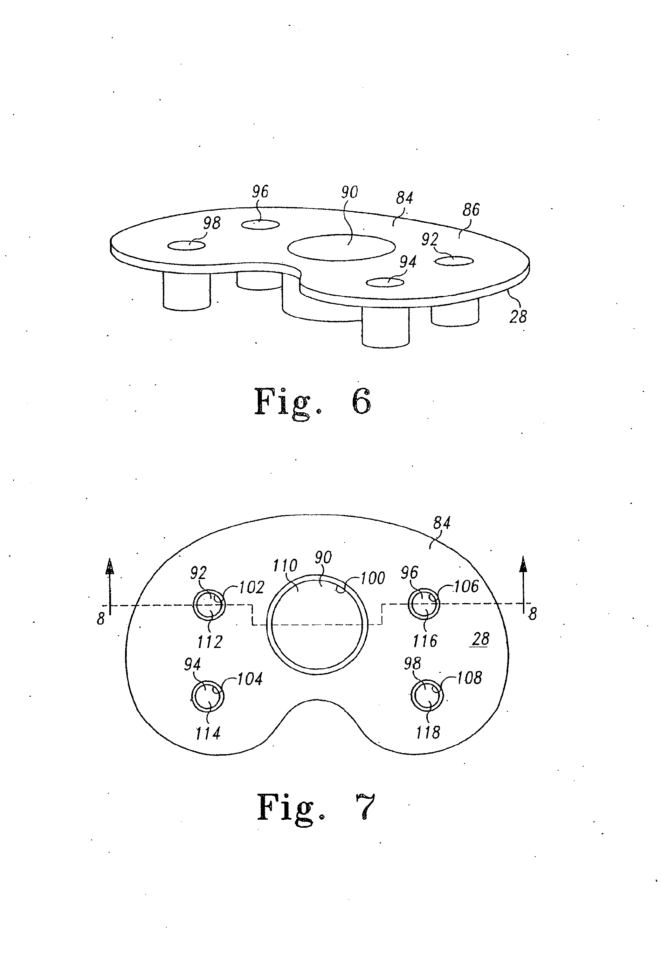

An embodiment of a first example of the porous portion 82 of a tibial tray 14, prior to being moulded to the solid polymer portion 80, is shown in FIGS. 6 to 8. This porous portion defines a base or preform 84 that has an upper surface 86 opposite from the distal surface 28. The upper surface 86 is generally flat and planar, and becomes the interface with the solid polymer portion 80 of the tray when the porous base or preform is moulded with the polymer to make the tibial tray 14. In this embodiment, the porous base or preform 84 also defines the proximal exterior surface 60, 62, 64, 66, 68 of each of the extensions 30, 32, 34, 36, 38. The proximal exterior surfaces 60, 62, 64, 66, 68 extend distally out from the bone-engaging distal surface 28 of the base or preform 84.

As shown in FIGS. 6 to 7, the upper surface 86 of the first porous base or preform 84 has a series of five holes or openings 90, 92, 94, 96, 98. These holes or openings correspond with the extensions 30, 32, 34, 36, 38.

The extensions 30, 32, 34, 36, 38 of the first embodiment define a stem 30 and four spaced pegs 32, 34, 36, 38. The stem 30 and pegs 32, 34, 36, 38 are configured to be implanted into a surgically prepared end of a patient's tibia (not shown).

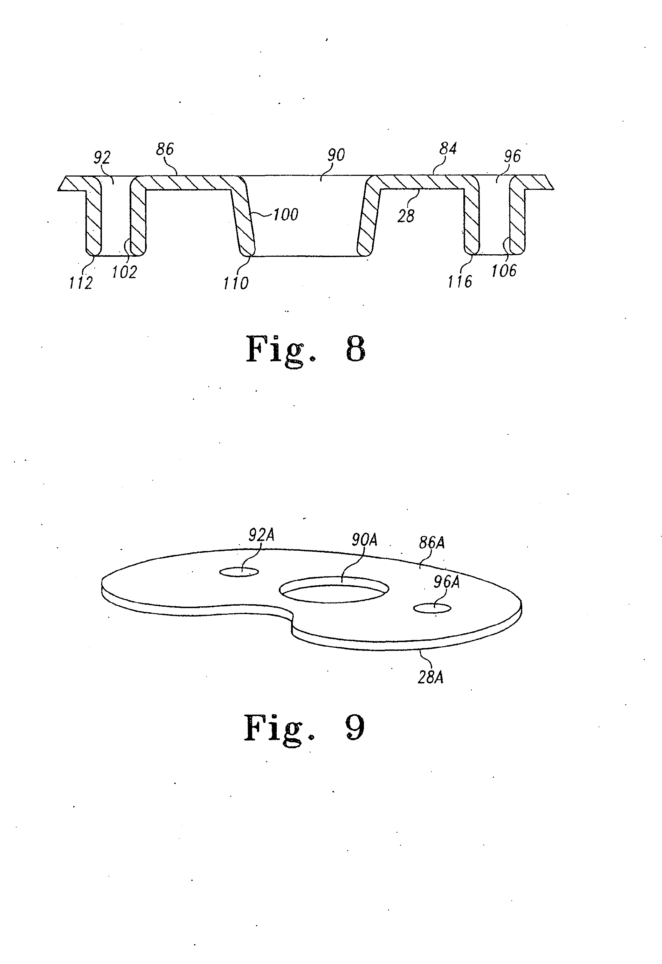

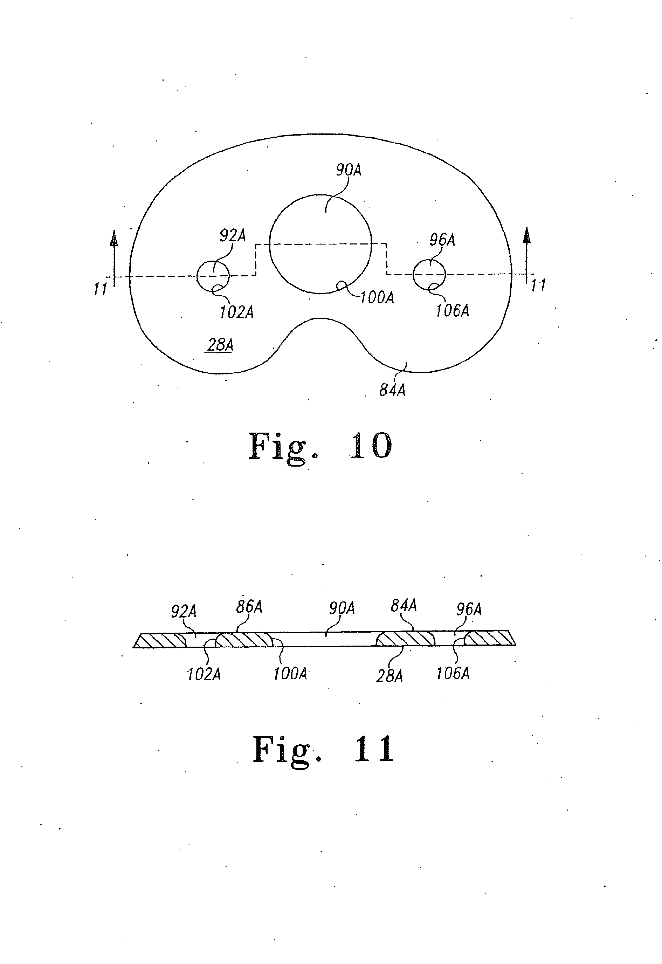

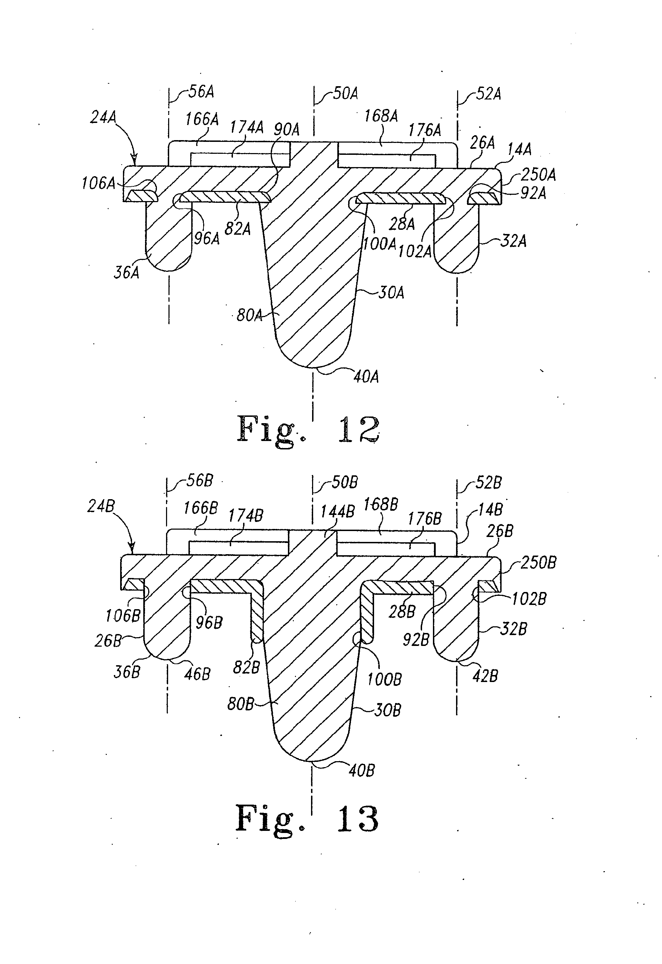

Additional embodiments of porous metal preforms 84A, 84B are illustrated in FIGS. 9 to 13. In these embodiments, the base or preform has three holes or openings 90A, 90B, 92A, 92B, 96A, 96B. A tibial tray made with from such a base or preform 84A, 84B, shown in FIGS. 12 and 13 at 14A and 14B, would have a stem 90A, 90B and two pegs 92A, 92B, 96A, 96B. The number and shapes of the extensions can be varied.

For the embodiments shown in FIGS. 9 to 13, like reference numerals have been used for parts corresponding with the first illustrated embodiment, followed by the letter "A" for the first alternative embodiment of FIGS. 9 to 12 and the letter "B" for the second alternative embodiment of FIG. 13. Generally, the description of the first embodiment applies to these alternative embodiments, except where distinctions are drawn.

As can be seen from a comparison of the preform 84 of the embodiment of FIGS. 6 to 8 to the preform 84A of the embodiment of FIGS. 9 to 11, the porous preform or base need not include any portions extending distally beyond the distal surface 28, 28A of the preform. A tibial tray formed from the preform 84A of FIGS. 9 to 11 would appear similar to the tray 14A shown in FIG. 12, in this embodiment, the porous portion 82A of the tray 14A does not define any portion of the extensions 30A, 32A, 34A, 36A, 38A, 40A, instead the porous portion 82A defines a portion of the platform 28A of the tibial tray only. The entire length of each of the extensions 30A, 32A, 34A, 36A, 38A, 40A in the embodiment of FIG. 12 comprise parts of the solid polymer portion 80A of the tibial tray 14A.

As can be seen from a comparison of the trays 14A and 14B of FIGS. 12 and 13, the extensions of the tibial tray need not all be the same. In the embodiment of FIG. 13, the central stem extension 30B of the tray 14B has a proximal exterior surface 60B that is part of the porous portion 82B of the tray 14B, with the distal exterior surface 70B being part of the polymer portion 80B of the tray 14B, the exterior surfaces of the other extensions 92B and 96B in the embodiment of FIG. 13 are part of the solid polymer portion of the tray 14B along their entire exposed length.

In each of the embodiments shown in the drawings, at least part of each extension 30, 32, 34, 36, 38, 30A, 32A, 36A, 30B, 32B, 36B is defined by the solid polymer portion 80, 80A, 80B of the tray 14, 14A, 14B. As shown in the cross-sections of FIGS. 5, 12 and 13, the solid polymer portion 80, 80A, 80B extends through the cavity defined by the interior surfaces 100, 102, 104, 106, 108, 100A, 102A, 106A, 100B, 102B, 106B at each hole 90, 92, 94, 96, 98, 90A, 92A, 96A, 90B, 92B, 96B and extends to the distal exterior surface 40, 42, 44, 46, 48, 40A, 42A, 46A, 40B, 42B, 46B of each extension 30, 32, 34, 36, 38, 30A, 32A, 36A, 30B, 32B, 36B.

The solid polymer portions 80, 80A, 80B of the illustrated tibial trays 14, 14A, 14B may comprise a reinforced bio-stable and biocompatible polymer, such as fibre-reinforced PEEK (polyetheretherketone). An example of a suitable material is PEEK reinforced with carbon fibres or glass fibres. A commercially available carbon fibre reinforced PEEK material is available from Invibio Inc. of West Conshohocken, Pennsylvania and Invibio Limited Corporation of Lancashire, United Kingdom (www.invibio.com) under the trade mark PEEK-OPTIMA. The carbon-reinforced PEEK supplied by Invibio is available with different concentrations of carbon fibre and Invibio reports different elastic moduli for the different concentrations: 20% carbon fibre by weight having an elastic or flexural modulus of 15 GPa, 30% carbon fibre by weight having an elastic or flexural modulus of 19 GPA, and 60% carbon fibre by weight having an elastic or flexural modulus of 50 GPa. It should be understood that the present invention is not limited to this particular PEEK material or to PEEK material for the polymer portion unless expressly called for in the claims. It is expected that other polyarylarylketone polymers, other fibre reinforced biocompatible polymeric materials and non-reinforced biocompatible polymeric materials are or will become available that will be appropriate for use in implementing the invention. In addition, it is expected that other fibres may be used for reinforcing the selected polymer, for example, the biocompatible polymer may be reinforced with hydroxyapatite (HA) whiskers of various volume fractions, as disclosed in US-A-2008/206297.

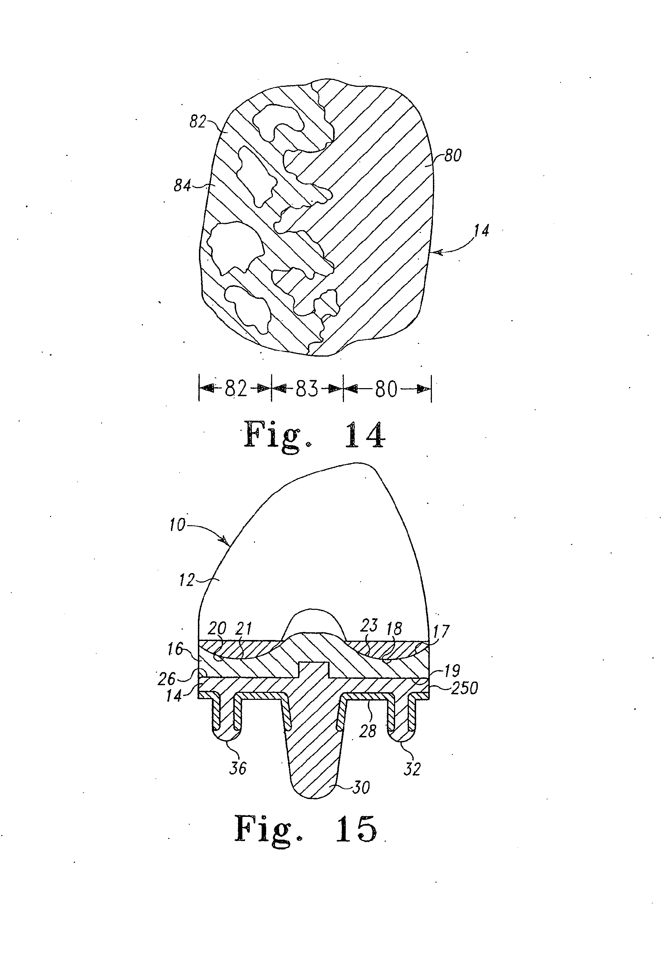

The porous portions 82, 82A, 82B of the tibial trays 14, 14A, 14B may comprise any commonly used biostable and biocompatible metal, such as stainless steel, titanium and standard cobalt chromium and titanium alloys, or may comprise a porous biocompatible polymer. Preferably, the porous portion 82, 82A, 82B has pores of such size, shape and number so that when the solid polymer portion 80, 80A, 80B of the tibial tray is moulded to the porous portion 82, 82A, 82B, an intermediate bonding layer is formed between the porous portion 82, 82A, 82B and the polymer portion 80, 80A, 80B. An example of such a structure is shown in FIG. 14, as shown in that drawing, there is a porous portion 82 that does not include any material of the solid polymeric portion 80, a solid polymeric portion 80 that does not include any material of the porous portion 82, and an intermediate portion 83 that includes material from both the polymeric portion 80 and the porous portion 82. In the intermediate portion 83, the polymeric material from the solid polymeric portion 80 and the skeleton from the porous portion 82 interdigitate to mechanically bond the solid polymeric portion 80 to the porous portion 82. This same interdigitation would be present in the embodiments of FIGS. 9 to 13 as well.

Various types of porous structure may be used for the porous portions 82, 82A, 82B of the tibial trays 14, 14A, 14B. For example, a titanium metal foam may be used, such as the foams disclosed in US-A-2008/199720, US-A-2010/098574, US-A-2009/326674 and US-A-2009/292365. Alternative materials are available. One example of a suitable alternative material is tantalum porous metal, disclosed, for example in US-5282861 and US-A-2009/192610. Another example of an alternative is a solid metal body made from an implantable metal such as stainless steel, cobalt chromium alloy, titanium, titanium alloy or the like and with a porous coating disposed on both the bone-engaging surface and the surface engaging the polymer portion of the tibial tray. One type of porous coating which may be used as the porous portions 82, 82A, 82B of the tibial trays 14, 14A, 14B is that used on implant components which are available from DePuy Orthopaedics Inc of Warsaw, Indiana under the trade mark Porocoat. The porous metal preform 84A may be made using any of the process described above or using other processes.

Porous biocompatible polymers are available as well. For example, US-A-2008/206297discloses porous biocompatible polymeric materials such as PEEK reinforced with hydroxyapatite fibres or whiskers and methods of making such materials. It is anticipated that such porous biocompatible polymers may be used for the porous portions 82, 82A, 82B of the tibial tray 14, 14A, 14B instead of metal.

Any biocompatible material having a surface of sufficient porosity and suitable mechanical properties to form a mechanical bond with the solid polymer portion 80, 80A, 80B when moulded together may be used as the porous portion 82, 82A, 82B of the tibial tray 14, 14A, 14B.

It may be desirable to incorporate additional materials into the porous portion 82, 82A, 82B of the tibial tray 14, 14A, 14B. For example, to facilitate bone ingrowth, a calcium phosphate such as hydroxyapatite may be coated or deposited on the porous portion 82, 82A, 82B, with or without other bioactive agents.

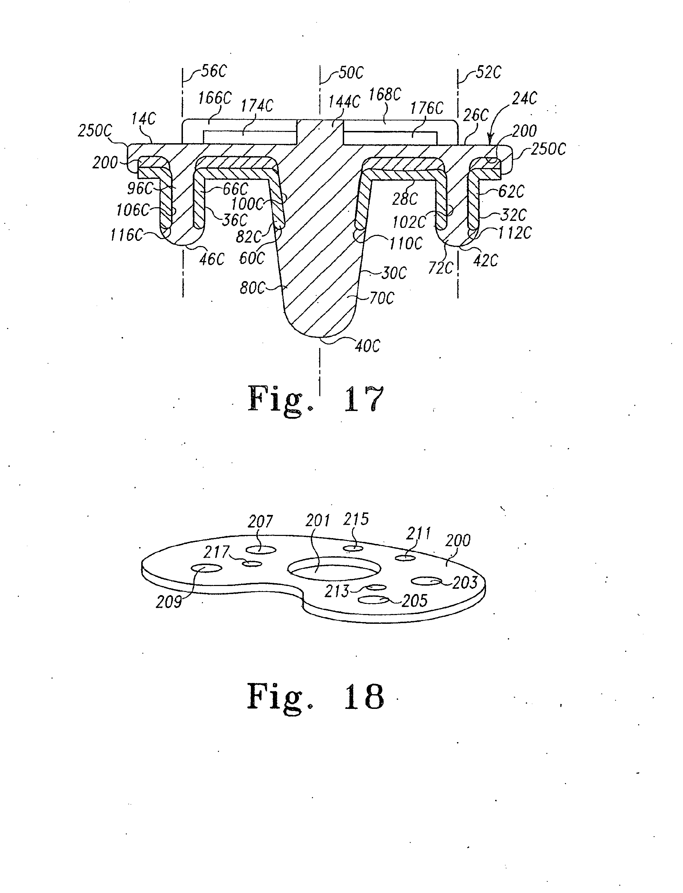

If the porous portion 82, 82A, 82B of the tibial tray 14, 14A, 14B is made of a polymeric material, it may be desirable to provide additional reinforcement to the tray. One way of providing such additional reinforcement is illustrated in the embodiment FIGS. 17 to 22. FIG. 17 is similar to FIG. 5, with the exception that a reinforcing plate 200 is added to the composite construct. For the embodiments shown in FIGS. 17 to 22, like reference numerals have been used for parts corresponding with the first illustrated embodiment, followed by the letter "C". Generally, the description of the first embodiment applies to these alternative embodiments, except where distinctions are drawn. In addition, it should be understood that the structures described below with respect to FIGS. 17 to 22 can be applied as well to the embodiments illustrated in FIGS. 9 to 13.

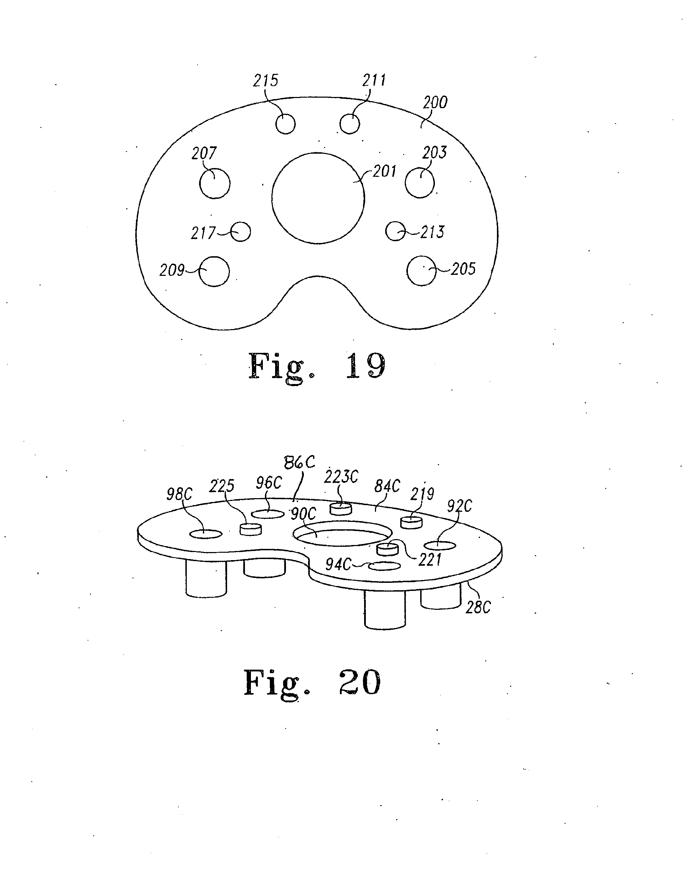

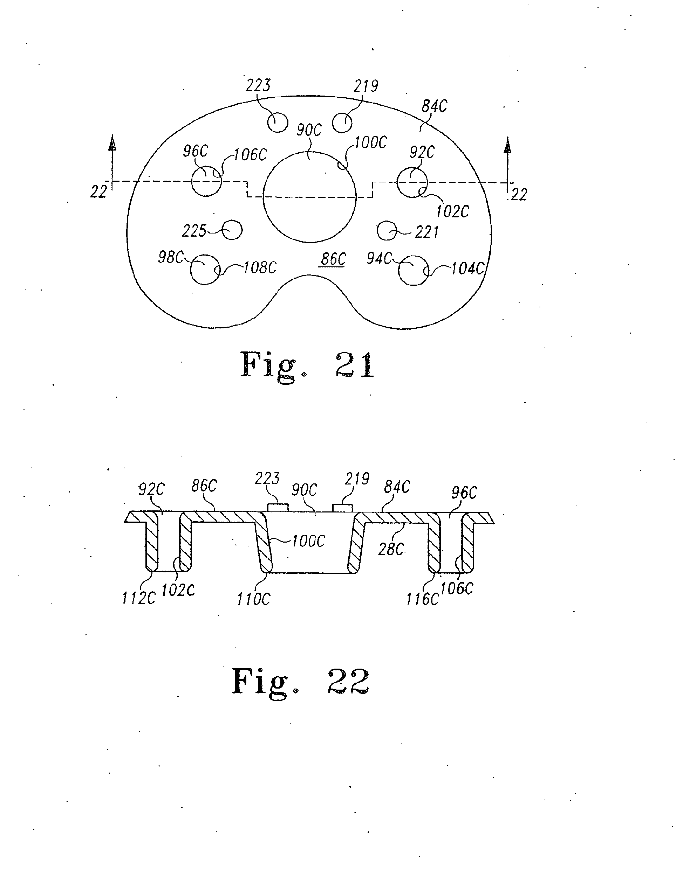

The reinforcing plate 200 in the embodiment of FIGS. 17 to 22 is positioned between portions of a porous preform 82C and the solid polymer portion 80C of the tray 14C. As shown in FIGS. 18 to 19, the plate 200 has an outline shaped generally the same as that of the tibial tray platform and includes a plurality of holes or bores. A large central through-bore 201 is sized, shaped and positioned to correspond with the bore 90C of the porous polymer preform 84C shown in FIGS. 20 and 21. The reinforcing plate 200 has four other spaced through-bores 203, 205, 207, 209 corresponding in size, shape and position to bores 92C, 94C, 96C, 98C of the porous polymer preform 84C. The reinforcing plate 200 also includes four spaced through-bores 211, 213, 215, 217 sized, shaped and positioned to fit over four raised abutments 219, 221, 223, 225 extending up from the upper surface 86C of the porous polymer preform 84C. It should be understood that the reinforcing plate 200 is not necessarily drawn to scale, the thickness of the plate 200 may be varied, along with the material, to achieve the desired properties for the entire tibial tray 14C. The plate 200 could be made out of metal, such as standard cobalt chromium alloy, stainless steel or titanium alloy. Alternatively, the plate 200 could comprise a biocompatible polymer, such as, for example, a fibre reinforced PEEK material or any of the other polymer materials described herein. For example, as discussed above, PEEK reinforced with carbon fibre is available in varying concentrations, one might select to make the reinforcing plate 200 out of a PEEK having one concentration of carbon fibre and make the solid polymer portion 80C out of PEEK having another concentration of carbon fibre. The material and dimensions (such as thickness) for the reinforcing plate 200 may be varied to provide a desired effective stiffness for the total tibial tray 14C.

All of the embodiments of tibial trays 14, 14A, 14B and 14C described above are suitable for supporting the bearing 16.

In a preferred embodiment, the bearing 16 is formed from a polymeric material, but comprises a different polymeric material from that used for the polymer portion of the tibial tray. Suitable polymeric materials for the bearing include ultrahigh molecular weight polyethylene (UHMWPE). The UHMWPE may comprise a cross-linked material, for example. Techniques for crosslinking, quenching, or otherwise preparing UHMWPE are disclosed in documents such as US-5728748, US-5879400, US-6017975, US-6242507, US-6316158, US-6228900, US-6245276 and US-6281264. The UHMWPE of the bearing material may be treated to stabilize any free radicals present therein, such as through the addition of an antioxidant such as vitamin E. Techniques for stabilizing UHMWPE with antioxidants are disclosed in, for example, US-A-2007/0293647 and US-A-2003/021216. It should be understood that the present invention is not limited to any particular UHMWPE material or to UHMWPE material for the bearing 16. It is expected that other materials for the bearing 16 are or will become available that will be useful in the invention.

Referring back to FIGS. 1 to 3, the first knee prosthesis is a fixed bearing prosthesis: the bearing 16 is secured to the tibial tray 14 through complementary locking features that eliminate or at least minimize any relative movement between the bearing 16 and the tibial tray 14 when these components are assembled.

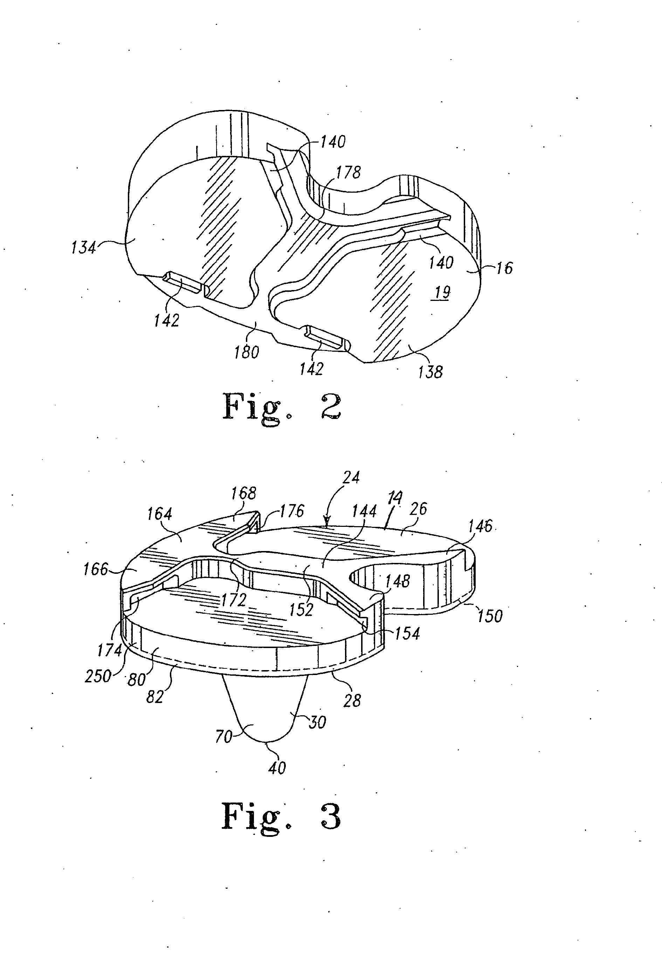

As shown in FIG. 2, the distal surface 19 of the bearing 16 includes a lateral pedestal 134 and a medial pedestal 138. The pedestals 134, 138 have posterior tabs 140 defined therein. A number of anterior tabs 142 are also defined in the bearing 16.

As shown in FIGS. 1 and 3, a generally Y-shaped posterior buttress 144 defines part the proximal surface 26 of the tibial tray 14. This posterior buttress 144 is formed in the polymer portion 80 of the tibial tray 14. In the first embodiment described herein, the posterior buttress 144 has a pair of arms 146, 148 extending along a posterior section of the perimeter of the proximal surface 26 of the tray's polymer portion 80. Specifically, the lateral arm 148 of the posterior buttress 144 extends along the posterior edge 150 on the lateral side of the polymer portion 80, whereas the medial arm 146 of the posterior buttress 144 extends along the posterior edge 150 on the medial side of the polymer portion 80 in a direction away from the lateral arm 148. A third arm 152 of the posterior buttress 144 extends anteriorly away from the intersection of the lateral arm 148 and the medial arm 146.

As shown in FIG. 1, the posterior buttress 144 has a pair of undercuts 154, 156 defined therein. Specifically, the lateral undercut 154 is defined in the lateral arm 148 of the posterior buttress 144, with the medial undercut 156 being defined in the medial arm 146 of the posterior buttress 144.

As also shown in FIGS. 1 and 3, a generally T-shaped anterior buttress 164 extends upwardly from the proximal surface 26 of the tibial tray 14. This anterior buttress 164 is formed in the polymer portion 80 of the tibial tray 14. In the first embodiment described herein, the anterior buttress 164 has a pair of arms 166, 168 extending along an anterior section of the perimeter of the proximal surface 26 of the tray's polymer portion 80. Specifically, the lateral arm 166 of the anterior buttress 164 extends along the anterior edge 170 on the lateral side of the polymer portion 80, whereas the medial arm 168 of the anterior buttress 164 extends along the anterior edge 170 on the medial side of the polymer portion 80 in a direction away from the lateral arm 166. A third arm 172 of the anterior buttress 164 extends posteriorly away from the intersection of the lateral arm 166 and the medial arm 168.

As shown in FIG. 3, the anterior buttress 164 has a pair of undercuts 174, 176 defined therein. Specifically, the lateral undercut 174 is defined in the lateral arm 166 of the anterior buttress 164, with the medial undercut 176 being defined in the medial arm 168 of the anterior buttress 164.

In the embodiment of FIGS. 1 and 3, the posterior buttress 144 of the tibial tray 14 is contiguous with the tray's anterior buttress 164. Specifically, the third arm 152 of the posterior buttress 144 is contiguous with the third arm 172 of the anterior buttress 164. However, other embodiments are contemplated, including arrangements in which the buttresses are not contiguous. Moreover, the two buttresses 144, 164 are herein described as being of a similar height, although the buttresses could be embodied as having dissimilar heights.

The two buttresses 144, 164 may be formed in the polymer material as part of a moulding process when the solid polymer portion 80 is moulded to the porous portion 82. Alternatively, the buttresses 144, 164 may be formed by machining the polymer portion 80 after moulding the solid polymer portion and the porous portion together. The undercuts 154, 156, 174, 176 may be formed in the polymer portion 80 by moulding, machining or other suitable process. Some combination of moulding and machining could also be used to form the buttresses 144, 164 and undercuts 154, 156, 174, 176.

To secure the tibial bearing 16 to the tibial tray 14, the posterior tabs 140 of the bearing 16 are positioned in the posterior undercuts 154, 156 of the tibial tray 14. The anterior portion of the tibial bearing 16 is then advanced downwardly toward the tibial tray 14 such that the anterior tabs 142 of the tibial bearing 16 are deflected by the anterior buttress 164 and thereafter snapped into the anterior undercuts 174, 176 of the anterior buttress thereby securing the bearing 16 to the tray 14.

As the anterior portion of the bearing 16 is advanced downwardly in such a manner, the buttresses 144, 164 of the tibial tray 14 are captured between the pedestals 134, 138 of the bearing's distal surface 19. Specifically, as shown in FIG. 2, the distal surface 19 of the bearing 16 has a posterior recess 178 and an anterior recess 180 defined therein. The posterior recess 178 is configured to compliment the shape of the posterior buttress 144 of the tibial tray 14. That is, when the bearing 16 is secured to the tibial tray 14, the sidewalls of the pedestals 134, 138 that define the posterior recess 178 contact the edges of the posterior buttress 144. Likewise, the anterior recess 180 is configured to compliment the shape of the anterior buttress 164 of the tibial tray 14, that is when the bearing 16 is secured to the tibial tray 14, the sidewalls of the pedestals 134, 138 that define the anterior recess 180 contact the edges of the anterior buttress 164. The dimensions of the recesses 178, 180 and the buttresses 144, 164 are selected such that a relatively tight fit is achieved. In such a way, the bearing 16 is fixed relative to the tibial tray 14. In particular, the configuration of the buttresses 144, 164 and the pedestals 134, 138 formed in the distal surface 19 of the bearing 16 prevent movement of the bearing 16 relative the tibial tray 14 in the anterior/posterior direction and the medial/lateral direction. Moreover, the posterior tabs positioned in the undercuts 154, 156 and the anterior tabs 142 positioned in the undercuts 174, 176 prevent lift off of the bearing 16 from the tibial tray 14. Rotational micromotion is reduced, if not prevented all together, by the relatively tight fit of the buttresses 144, 164 of the tibial tray 14 into the recesses 178, 180 of the bearing 16, particularly along the third arm 152 of the posterior buttress 144 and/or the third arm 172 of the anterior buttress 164.

A given design of a fixed-bearing knee prosthesis is typically made commercially available in a variety of different sizes, particularly in a variety of different widths. This is done to accommodate the many variations in patient size and anatomy across a population. However, the configuration of the fixed-knee prosthesis 10 of the present disclosure allows for a high degree of flexibility in regard to the sizing of the tibial tray 14 and the bearing 16. Each of the individual trays 14 having a size (e.g., width) that is different from the other trays 14 of the group, the basic configuration of the posterior buttress 144 and the anterior buttress 164 remains the same across the range of differently-sized trays 14. Specifically, the location of the undercuts 154, 156 defined in posterior buttress 144, respectively, remains the same across the range of differently-sized trays 14. Even though the posterior undercuts 154, 156 remain in the same location across the range of differently-sized trays 14, the width of the arms 146, 148 is varied to accommodate the overall width of a given tray 14. In a similar manner, the location of the undercuts 174, 176 defined in anterior buttress 164, respectively, remains the same across the range of differently-sized trays 14, although the width of the arms 166, 168 is varied to accommodate the overall width of a given tray 14. The size and configuration of the third arms 152, 172 of the posterior buttress 144 and the anterior buttress 164, respectively, remain unchanged across the range of differently-sized trays 14.

Differently-sized bearings 16 may also be configured in such a manner. In particular, a plurality of the bearings 16 may be designed with each of such a plurality of bearings 16 having a different size, particularly a different width. However, each of such differently-sized bearings 16 may include mating features that are commonly-sized and commonly-located with the commonly-sized and commonly-located features of the tibial tray 14 described above. In particular, each of the bearings 16 across a range of differently-sized bearings may include a posterior recess 178 and an anterior recess 180 that is positioned and sized to tightly fit against the edges of the posterior buttress 144 and the anterior buttress 164, respectively, of each of the tibial trays 14 across the range of differently-sized trays 14.

The posterior tabs 140 are commonly-sized and commonly-located across the range of differently-sized bearings 16 so that they are positioned in the respective posterior undercuts 154, 156 of each of the tibial trays 14 across the range of differently-sized trays 14. Likewise, the anterior tabs 142 are commonly-sized and commonly-located across the range of differently-sized bearings 16 so that they are positioned in the respective anterior undercuts 174, 176 of each of the tibial trays 14 across the range of differently-sized trays 14.

It should be appreciated from the above-discussion that the general configuration of the buttresses 144, 164 (including contiguous variations thereof) is the same across a range of differently-sized tibial trays 14. Likewise, the general configuration of the recesses 178, 180 (including contiguous variations thereof) and the general configuration of tabs 140, 142 are the same across a range of differently-sized bearings 16. As such, a number of differently-sized bearings 16 may be secured to a given tibial tray 14. This provides the orthopaedic surgeon with greater flexibility of matching the knee prosthesis 10 to a particular patient's anatomy.

Other configurations of the posterior buttress 144 and the anterior buttress 164 are also contemplated, as well as other configurations of locking mechanisms. Details of locking mechanisms for tibial trays and bearings for fixed bearing applications are disclosed in US-7628818 and US-A-2009/0082873.

To make the tibial tray 14, 14A, 14B, 14C, the porous base or preform 84, 84A, 84B, 84C is first made, using any suitable process for example as described above. The preform 84, 84A, 84B, 84C may be placed in a suitable moulding apparatus and the material for the solid polymer portion 80, 80A, 80B, 80C (such as fibre-reinforced PEEK) then moulded onto the preform. Injection moulding, for example, may be used.

During the moulding process, some of the polymer flows over the proximal surface 86, 86A, 86B of the porous base or preform 84, 84A, 84B and into the holes 90, 92, 94, 96, 98, 90A, 92A, 94A, 96A, 98A, 90B, 92B, 94B, 96B, 98B of the preform. In the case of the first embodiment, the polymer flows through the holes and into the channels defined by the interior surfaces 100, 102, 104, 106, 108 of the extensions 30, 32, 34, 36, 38, and out of the holes 110, 112, 114, 116, 118 at the distal ends of the porous portions of the extensions to form the polymeric distal ends 40, 42, 44, 46, 48 and distal exterior surfaces 70, 72, 74, 76, 78 of the extensions 30, 32, 34, 36, 38. In the case of the fourth embodiment, during the moulding process polymer flows over the upper surface of metal plate 200 and through the through holes of the plate 201, 203, 205, 207, 209 and into the through holes 90C, 92C, 94C, 96C, 98C of the porous preform 84C and out of holes at the distal ends of the porous portions of the extensions to form the polymeric distal ends 40C, 42C, 44C, 46C, 46D, 46E and distal exterior surfaces of the extensions 30C, 32C, 34C, 34D, 34E. As discussed in more detail below, during the moulding process, polymer also flows through the through holes 211, 213, 215, 217 of the plate 200 and into the pores of the porous polymer of the four raised abutments 219, 221, 223, 225.

Along the interfaces of the polymer and the porous preform, some of the polymer flows into some of the pores of the preform 84, 84A, 84B, producing a structure such as that shown schematically in FIG. 14, where a region with interdigitated polymer and porous material is formed to bind the polymer portion 80 to the porous base or preform 84. In the case of the fourth embodiment, the polymer will flow into the pores of the abutments 219, 221, 223, 225 and interdigitate as shown in FIG. 14 to bond the two polymer portions 80C, 82C together, with the reinforcing plate 200 held between them. It should be understood that use of the four porous abutments 219, 221, 223, 225 represents one method of bonding the solid polymer portion 80C to the porous polymer portion 82C, differing numbers, sizes and shapes of areas in the plate 200 that allow for interdigitation of the two polymers may be used, for example.

As indicated above, the locking features (such as the buttress 144 and undercuts 154, 156, 174, 176) may be moulded and/or machined or otherwise finished to form the mounting surface 26, 26A, 26B, 26C of the tibial tray 14, 14A, 14B, 14C.

Production cost for such a tibial tray is expected to be lower than the cost of producing a comparably sized tibial tray made completely of metal.

The composite tibial tray 14, 14A, 14B, 14C so manufactured can be expected to have advantageous properties. For example, such a tibial tray would be expected to have an effective stiffness (stiffness of the composite construct including both the porous portion 82 and the polymer portion 80) lower than that of a similarly sized and shaped tibial tray made solely of standard biocompatible metal alloys. With such an effective stiffness, it is anticipated that the tibial tray of the present invention would provide optimum load transfer to the underlying bone to minimize or prevent stress shielding and the resultant bone loss.

Typically a plurality of such tibial trays of various sizes would be included in a kit, along with a plurality of sizes of femoral components and bearings. The surgeon or operating room staff would then select the appropriate size of tibial tray and bearing, and assemble them into a structure such as that shown in FIG. 15. This assembly may be made either before or after the tibial tray 14, 14A, 14B, 14C is implanted on the prepared tibia. When so assembled, the distal mounting surface 19 of the bearing 16 contacts and bears against the proximal mounting surface 26, 26A, 26B, 26C of the tibial tray 14, 14A, 14B, 14C. A suitable femoral component 12 would be implanted, and would articulate with the bearing 16 as shown in FIG. 15. Notably, the tibial tray 14, 14A, 14B, 14C is suitable for cementless implantation.

After implantation, it is anticipated that bone will grow into the porous portion 82, 82A, 82B, 82C of the tibial tray 14, 14A, 14B, 14C. Bone will not, however, grow into the exposed solid polymer portion 80, 80A, 80B, 80C of the tibial tray 14, 14A, 14B, 14C. Thus, it is anticipated that there will be bone ingrowth into the distal surface 28, 28A, 28B, 28C of the tibial platform 24, 24A, 24B, 24C. In addition, for the first illustrated embodiment, bone ingrowth is also anticipated into the proximal exterior surfaces 60, 62, 64, 66, 68 of the extensions 30, 32, 34, 36, 38 adjacent to the distal surface 28 of the tibial platform 24. A similar result is expected for the exterior surfaces 60C, 62C, 66C of the extensions 30C, 32C, 36C and distal surface 28C illustrated in FIG. 17. Radial pressure along the proximal exterior surfaces 60, 62, 64, 66, 68, 60C, 62C, 66C is expected to be uniform, to stimulate bone ingrowth in all directions on the stem and pegs 30, 32, 34, 36, 38, 30C, 32C, 36C. Bone will not, however, grow into the exposed polymer portions of the distal exterior surfaces 70, 72, 74, 76, 78, 70C, 72C, 76C at the distal ends 40, 42, 44, 46, 48, 40C, 42C, 46C of the extensions 30, 32, 34, 36, 38, 30C, 32C, 36C. Thus, bone ingrowth should occur at the proximal end of the tibial tray but not at the distal end. In addition, in each illustrated embodiment, the interfaces of the porous portion 82, 82A, 82B, 82C of the tray 14, 14A, 14B, 14C and the bone are all readily accessible from the tibial plateau.

The central stem 30, 30A, 30B, 30C is expected to provide stability against lift off for the tibial tray. The pegs 32, 34, 36, 38, 32A, 36A, 32B, 36B, 32C, 36C surrounding the central stem 30, 30A, 30B, 30C are expected to reduce shear and micromotion proximally, especially after bone ingrowth has occurred.

If it becomes necessary to remove the tibial tray 14, 14A, 14B, 14C the surgeon may cut along the distal surface 28, 28A, 28B, 28C of the tibial tray platform 24, 24A, 24B, 24C\ to sever the connection between the patient's bone and the tibial tray platform 24, 24A, 24B, 24C. If the porous portion 82, 82A, 82B, 82C of the tibial tray 14, 14A, 14B, 14C is made of a material such as a metal or polymer foam, the surgeon may also cut through all of the extensions 30, 32, 34, 36, 38, 30A, 32A, 36A, 30B, 32B, 36B, 30C, 32C, 36C at the junctures of the extensions and the distal surface 28, 28A, 28B, 28C of the tibial platform 24, 24A, 24B, 24C and easily remove the tibial platform 24, 24A, 24B, 24C. If the tibial tray 14, 14A, 14B, 14C is similar to the first and fourth illustrated embodiments, the surgeon may then cut around the outer perimeter of each extension 30, 32, 34, 36, 38, 30C, 32C, 36C to sever the connection between the bone and the porous proximal external surfaces 60, 62, 64, 66, 68, 60C, 62C, 66C of the extensions 30, 32, 34, 36, 38, 30C, 32C, 36C. Each extension may then be readily removed. Notably, since no bone ingrowth has occurred at the distal ends of the extensions, the amount of bone that needs to be resected should be substantially less compared to a system that uses fully porous stems and pegs.

Thus, the present invention provide a knee prosthesis with a modular tibial implant component suitable for cementless fixation. The tibial implant component can be readily removed from the bone in revision surgery to conserve native bone. The tibial implant can also have a modulus of elasticity less than that of conventional solid titanium and cobalt chromium alloy trays.

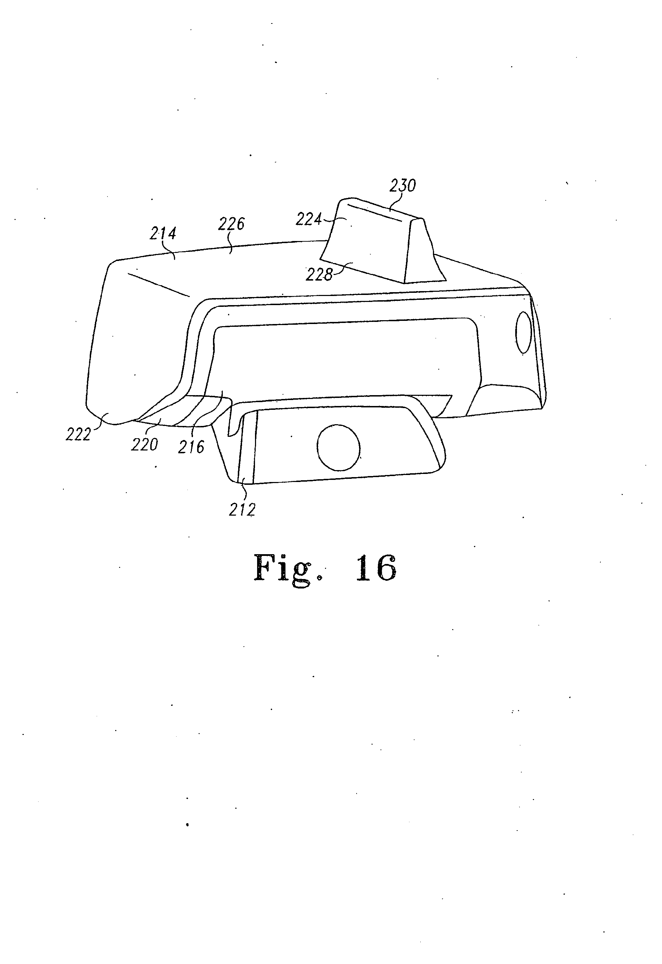

The invention is applicable to other joint prostheses as well. An example of such a joint prosthesis is an ankle prosthesis as shown in FIG. 16, which comprises a talar component 212, a composite distal tibial component 214 and a bearing 216. The composite distal tibial component 214 comprises a distal solid polymer portion 220 and a proximal porous portion 222, bonded together as described above for the knee prosthesis 10. As in the knee prosthesis 10, the solid polymer portion 220 and the bearing have mounting surfaces with complementary locking features (not shown) so that the bearing 216 can be fixed to the tibial component 214. The illustrated distal tibial component 214 has a proximal extension 224 extending proximally from the bone-engaging surface 226 of the tibial component 214. The proximal extension 224 may provide polymeric outer surfaces for engaging the bone or the distal portion 228 may comprise porous material and the proximal portion 230 comprise solid polymer.

Implant components discussed above might have surfaces that do not contact bone or another part of the implant component. For example, in the embodiments of FIGS. 1, 3, 5, 12, 13 and 17, peripheral surface 250, 250A, 250B, 250C of the tibial tray 14, 14A, 14B, 14C extends between the bone-engaging surface 28, 28A, 28B, 28C and the proximal mounting surface 26, 26A, 26B, 26C. To prevent soft tissue irritation, this peripheral surface 250, 250A and 250B in the embodiments of FIGS. 1, 3, 5, 12 and 13 is part of the solid polymer portion 80, 80A, 80B of the tibial tray 14, 14A, 14B, thus, soft tissue will not be irritated through contact with the porous portion 82, 82A, 82B of the tibial tray 14, 14A, 14B. In the embodiment of FIG. 17, the porous portion 82C has reduced outer dimensions compared to the solid polymer portion 80C to prevent contact with soft tissue. Essentially, a substantial part of the porous portion 82C fits within a pocket in the solid polymer portion 80C, while a part of the porous portion 82C stands proud to thereby ensure that the surface 28C fully engages and transfers load to the underlying bone.

## Claims

1. An ankle knee prosthesis comprising:
a distal tibial component having a (i) proximal bone-engaging surface, (ii) a distal mounting surface, and (iii) an extension extending from the proximal bone-engaging surface to a proximal end along an axis intersecting the proximal surface, the extension having an axial length and an exterior surface including a distal exterior surface adjacent to the proximal bone-engaging surface of the distal tibial component and a proximal exterior surface, the proximal exterior surface proximally extending from the proximal end for at least part of the axial length of the extension,
a bearing having an articulation surface and a mounting surface, and
a talar component having an articulation surface and a bone-engaging surface,
in which:
the distal tibial component comprises a composite including a solid polymer portion and a porous portion,
the solid polymer portion of the distal tibial component defines the proximal end of the extension and the mounting surface of the distal tibial component and bears against the mounting surface of the bearing,
the solid polymer portion of the distal tibial component is secured to the porous portion of the tibial component, and
the porous portion of the tibial component defines the proximal bone-engaging surface of the tibial component and the proximal exterior surface of the extension.

2. The ankle prosthesis of claim 1 in which the porous portion defines the distal exterior surface of the extension.

3. The ankle prosthesis of claim 1 in which the solid polymer portion comprises polyetheretherketone (PEEK).

4. The ankle prosthesis of claim 3 in which the solid polymer portion comprises fibre-reinforced PEEK.

5. The ankle prosthesis of claim 1 in which the bearing comprises a polymer material different from the solid polymer portion of the distal tibial component.

6. The ankle prosthesis of claim 5 in which the bearing comprises UHMWPE and the solid polymer portion of the distal tibial component comprises fibre-reinforced PEEK.
